# EUROPÄISCHE PATENTSCHRIFT

(11) **EP 1 264 014 B1**
(45) Veröffentlichungstag und Bekanntmachung des Hinweises auf die Patenterteilung: **05.10.2005**
(21) Anmeldenummer: 01946911.3
(22) Anmeldetag: 25.01.2001
(51) Int. Cl.: D01F 2/28, D01F 9/00, D01D 5/28, D01D 5/40, A61L 15/22

(54) **POLYELEKTROLYT-FESTSTOFFSYSTEM, VERFAHREN ZUR HERSTELLUNG DESSELBEN SOWIE WUNDVERBAND**
POLYELECTROLYTE SOLID SYSTEM, METHOD FOR THE PRODUCTION THEREOF AND A WOUND DRESSING
SYSTEME DE POLYELECTROLYTE SOLIDE, PROCEDE PERMETTANT DE LE PRODUIRE ET PANSEMENT

(30) Priorität: 27.01.2000 DE 10003397
(43) Veröffentlichungstag der Anmeldung: 11.12.2002
(73) Patentinhaber: Paul Hartmann Aktiengesellschaft, 89522 Heidenheim (DE)
(72) Erfinder: EFFING, Jochem, 89542 Herbrechtingen (DE); RIEDEL, Bernd, 07333 Unterwellenborn (DE)
(74) Vertreter: Langöhrig, Angelika Beate
(86) Internationale Anmeldenummer: PCT/EP2001/000785
(87) Internationale Veröffentlichungsnummer: WO 2001/055486

(56) Entgegenhaltungen:
- DE-A- 4 120 053
- DE-C- 19 741 063
- RIEDEL B ET AL: "NOVEL POLYANION-POLYCATION-MICROFIBRIDE BLEND NONWOVENS BASED ON CELLULOSE DERIVATIVES" CHEMICAL FIBERS INTERNATIONAL,DEUTSCHER FACHVERLAG,DE, Bd. 49, Nr. 1, März 1999 (1999-03), Seiten 55-57, XP000827376 ISSN: 0340-3343

## Beschreibung

Gegenstand der Erfindung ist ein Polyelektrolyt-Feststoffsystem, ein Verfahren zu seiner Herstellung sowie ein Wundverband.

Polyelektrolyte (PEL) besitzen aufgrund ihres hohen Gehaltes kovalent gebundener Salzgruppen die Fähigkeit, große Mengen Wasser aufzunehmen und dabei in Lösung zu gehen. In vielen Anwendungsfällen, insbesondere im Bereich der Wundbehandlung, der Kinderpflege und der Inkontinenzmaterialien, in denen es gilt, abgesondertes Wasser bzw. wässriges Sekret zu binden, ist es erforderlich, die Integrität des verwendeten Absorbers zu erhalten, damit er nach Gebrauch in einer mechanisch stabilen Form entfernt werden kann. Um dies zu gewährleisten, werden die ansonsten wasserlöslichen Polyelektrolyte auf verschiedenste Weise über Nebenvalenz-Bindungen, durch kovalente oder ionische Bindung so weit vernetzt, dass bei Wasserzutritt die Solvatation nur bis zum Stadium hochgequollener Gele erfolgt.

Da solche Absorber auf Polyelektrolyt-Basis aufgrund ihres hohen Salzgehaltes und ihrer Vernetzung im festen Zustand hart und spröde sind, werden sie, um eine gute Handhabbarkeit zu erreichen und um sie ausspülsicher zu fixieren, in flexible Polymere, insbesondere in Cellulose oder in Polyurethane eingelagert.

So beschreibt die Firma Beiersdorf unter dem Markennamen "Cutinova" einen Polyurethanschaum, in welchem feinste Superabsorberpartikel aus partiell vernetzten Polyelektrolytpulvern eingelagert sind. Dieses elastische Material ist in der Lage, in physiologische Kochsalzlöschung eingebracht, erhebliche Mengen Wasser aufzunehmen und als saugfähige Auflage für das feuchte Wundmanagement zu dienen.

Weiterhin ist es bekannt, Na-Carboxymethylcellulose als Polyelektrolyt (PEL) gemeinsam mit Cellulose nach dem Viskoseverfahren zu hochquellfähigen Viskosefasern zu verarbeiten. Die Fixierung der an sich wasserlöslichen Na-Carboxymethylcellulose in den Viskosefäden wird dabei durch die Ausbildung von durch Wasser nicht lösbaren intermolekularen Wasserstoffbrückenbindungen zwischen den Makromolekülen der Cellulose und denjenigen der Na-Carboxymethylcellulose erreicht. Nachteilig ist auch hier, daß die Polyelektrolyte in ein zweites flexibles Polymer eingebettet werden müssen.

Des weiteren wird in EP 0 616 650 ein Verfahren beschrieben, bei dem polyelektrolythaltige, hochquellfähige Fäden aufwendig und deshalb nachteilig durch partielle chemische Umwandlung von Cellulosefasern, insbesondere von Lyocellfasern, erzeugt werden können.

Ionisch vernetzte Polyelektrolyte, insbesondere ionisch hochvernetzte Polyelektrolytkomplexe, auch Polyanion-/Polykationkomplexe genannt, wurden trotz ihrer leichten Zugänglichkeit bisher nur in geringem Umfang für die Herstellung von absorbierenden Materialien verwendet. Polyelektrolytkomplexe entstehen durch die Reaktion anionischer und kationischer Makromoleküle (Polyelektrolyte) und sind über eine Vielzahl von Salzgruppen ionisch miteinander vernetzt. Sie sind in komplizierten Lösungssystemen, insbesondere Salzlösungen (JP 49099651), Ameisensäure (JP 62183768) oder Wasser/HCl/Dioxan (JP 49010232) löslich. Aus diesen Lösungen lassen sich Membranen herstellen, die, wie in US-PS 3,546,142 und US-PS 3,549,016 beschrieben, für die Ultrafiltration in wässrigen Lösungen geeignet sind. In diesen Polyelektrolytkomplex-Membranen sind die Makromoleküle strukturlos in einer indifferenten Wirrlage angeordnet.

Dagegen zeichnen sich die Polyelektrolytkomplex-Membranen, die gemäß DD-PS 160 393 und DD-PS 218 734/A4 an den Berührungsflächen von Lösungen anionischer und kationischer Polyelektrolyte entstehen, durch einen vorteilhafteren Bi-Layeraufbau aus. Jedoch sind diese Polyelektrolytkomplex-Membranen sehr dünn und deshalb mechanisch nur wenig belastbar. Sie werden neben der Ultrafiltration (DD-PS 152 287) vor allem zum Verkapseln biologisch aktiver Materialien vorgeschlagen (DD-PS 200 471/3; DD-PS 219 795/A1).

DE 197 41 063 beschreibt Flächengebilde in Form von Papier, Vlies, Gewebe oder Laminat, die aus einer Mischung feiner, in Wasser löslicher oder stark quellbarer anionischer und kationischer polyelektrolythaltiger Fasern und/oder Fibrids und/oder sphärischer Partikel hergestellt werden. Die endgültige Struktur dieser Materialien bildet sich erst nach Einwirkung von Wasser oder wässrigen Lösungen und/oder wässrigen Emulsionen und/oder wässrigen Suspensionen, durch die an den Berührungsflächen zwischen den polyanionischen und polykationischen Bestandteilen entstehenden Polyelektrolytkomplex-Membranen. Nachteilig ist hierbei, daß die entgegengesetzt geladenen Fasern, Fibride oder sphärische Partikel dabei nur punktuell und oberflächlich ionisch vernetzt und deshalb das entstandene Gel eine für den Gebrauch nur unzureichende mechanische Stabilität aufweist.

Weiterhin wurden in den letzten zwei Jahrzehnten große Fortschritte in der Behandlung von dermalen Wunden, wie z. B. von Verbrennungen, Operationswunden, Ulcera gemacht. Insbesondere haben die sogenannten "hydroaktiven Wundverbände" an Bedeutung gewonnen. Diese Verbände ermöglichen es, die Wunde feucht zu halten und gleichzeitig überschüssiges Sekret aufzusaugen, das Eindringen von Bakterien von außen zu verhindern sowie einen atraumatischen Verbandwechsel durch das Nichtverkleben mit der Wunde durchzuführen.

Erstrebenswert sind allgemein für die ersten Phasen der Wundheilung, in denen eine Reinigung erfolgt und Sekret vorliegt, solche Wundauflagen, die sich sehr gut dem Wundgrund anpassen lassen bzw. leicht in tiefere Wunden einzutamponieren sind, eine hohe Sekretaufnahmefähigkeit unter Ausbildung eines Gels besitzen und im gequollenen Zustand eine ausreichend hohe Konsistenz besitzen, so dass sie in einem oder wenigen Stücken aus der Wunde entfernt werden können. In späteren Phasen der Wundheilung sind oftmals Wundauflagen von Vorteil, die Feuchtigkeit an die Wunde abgeben und diese vor dem Austrocknen bewahren.

Von größter Bedeutung in der Praxis sind heute Verwendungen auf der Basis von Hydrokolloiden, Hydrogelen und Faserverbänden aus Alginaten.

Hydrokolloidverbände sind Verbände, in denen Hydrokolloide (wasserabsorbierende Partikel bzw. Polymere auf Polyelektrolytbasis) in einer Elastomermatrix dispergiert sind. Sie haben eine hohe Aufnahmefähigkeit für Sekret und sind selbsthaftend. Von Nachteil ist die mangelnde Tamponierbarkeit in tiefere bzw. zerklüftete Wunden sowie die Neigung, bei hoher Sekretaufnahme partiell zu zerfallen, wodurch der Reinigungsaufwand beim Verbandwechsel erhöht wird. Die Anwendung bei infizierten Wunden ist durch die Semiokklusivität der Verbände problematisch.

Hydrogele als Wundverbände sind durch ihr geringes Sekretaufnahmevermögen in ihrer Anwendung auf mittel bis schwach sezernierende Wunden beschränkt. Viele der Gele zeichnen sich zudem durch eine starke Verflüssigung aus, so dass sie durch intensives Spülen beim Verbandwechsel aus der Wunde entfernt werden müssen. Hydrogele können aber bei trockenen Wunden als feuchtigkeitsspendende Verbände eingesetzt werden.

Wundauflagen auf der Basis von Alginatfasern in Form von Kompressen bzw. Tamponatstreifen lassen sich zwar sehr gut in tiefe und komplexe Wunden eintamponieren und sind bei der Wundreinigung sehr effektiv, jedoch bildet sich bei der Sektretaufnahme ein recht flüssiges Gel, das durch intensives Spülen beim Verbandwechsel aus der Wunde entfernt werden muss.

Diese Materialien erfüllen die oben beschriebenen Leistungseigenschaften nur begrenzt, da sie entweder bei starker Sekretaufnahme ihre Stabilität verlieren bzw. verflüssigen oder schlecht zu tamponieren sind und bei infizierten Wunden nur eingeschränkt verwendbar sind.

Es ist daher Aufgabe der Erfindung, ein Polyelektrolyt-Feststoffsystem bzw. ein Verfahren zur Herstellung desselben bereitzustellen, das eine ausreichende mechanische Stabilität aufweist. Darüber hinaus soll ein Wundverband bereitgestellt werden, der aus einem tamponierfähigen Material mit hoher Saugfähigkeit besteht, das im gequollenen Zustand einen hohen inneren Zusammenhalt besitzt, so dass die Wundauflage beim Verbandwechsel in einem oder wenigen Stücken aus der Wunde entfernt werden kann, wobei die Wundheilung aktiv unterstützt wird.

Überraschenderweise konnte gefunden werden, dass ein Polyelektrolyt-Feststoffsystem insbesondere in Form von Flocken, Flockenverbunden, Vliesen oder Papier, wobei dieses eine Mischung aus faserförmigen Gebilden, die neben einem ionisch vernetzten Kern oder Block noch polyanion- und/oder polykationhaltige Bestandteile enthalten, und aus polyanion-und/oder polykationhaltigen Gebilden umfasst, wobei die jeweiligen anionischen und kationischen Polyelektrolyte wasserlösliche Polyelektrolyte sind, die Polymerketten mit mindestens 10 Monomerbausteinen besitzen und wobei das Verhältnis der polyanionischen und der polykationischen Bestandteile in dem Gemisch der faserförmigen polyanionpolykatkion-komplexhaltigen Gebilde und der polyanion-und/oder polykation-haltigen Gebilde bis zu 98 % vom stöchiometrischen Verhältnis Polyanion zu Polykation abweicht, diese Nachteile nicht aufweist, sondern bei Zutritt von Wasser oder wässrigen Lösungen, wie z. B. einer 0,9%-igen Kochsalzlösung oder wässrigen Sekreten, unter Wahrung seiner Integrität zu mechanisch stabilen Gelen aufquillt. Bei Einwirkung von Wasser vernetzen die Gebilde miteinander über ionische Bindungen und es kommt zur Ausbildung hoch gequollener, mechanisch stabiler Gele. Aufgrund der saugenden und gelbildenden Eigenschaften können die Materialien vielseitig verwendet werden, z. B. als Wundauflagen, medizinische Implantate oder Deposite, in der Kosmetik als Feuchtigkeitsspender, als Trägermaterial für Zellkulturen sowie als Überzüge, Dichtungsmaterialien oder als Bindemittel.

Wesentliches Merkmal der erfindungsgemäßen Polyelektrolyt-Feststoffsysteme sind die faserförmigen polyanion-polykation-komplexhaltigen Gebilde. Ein solches Gebilde enthält neben dem ionisch vernetzten Kern bzw. Block noch polyanionische und/oder polykationische Bestandteile. Die morphologisch den Fibriden zuzuordnenden, faserförmigen polyanion-polykation-komplexhaltigen Gebilde können die nachfolgende schematisch dargestellte Struktur aufweisen:

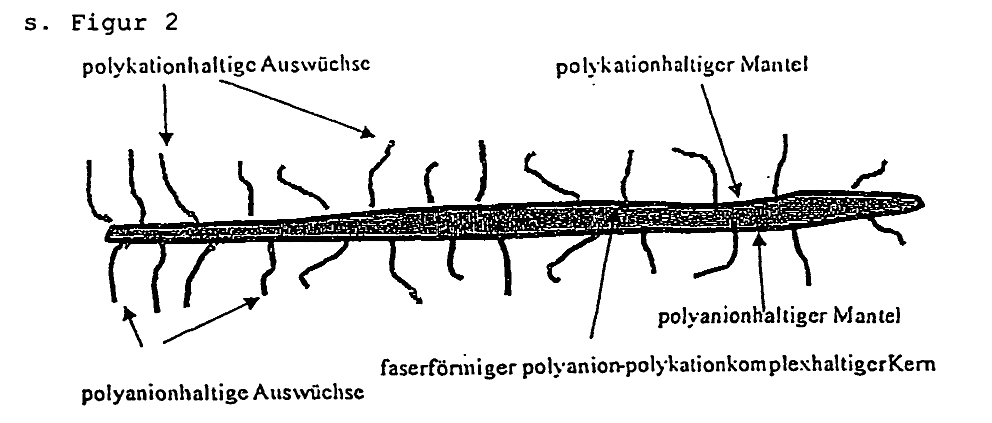

Sie können aber auch den nachfolgend skizzierten Blockaufbau besitzen:

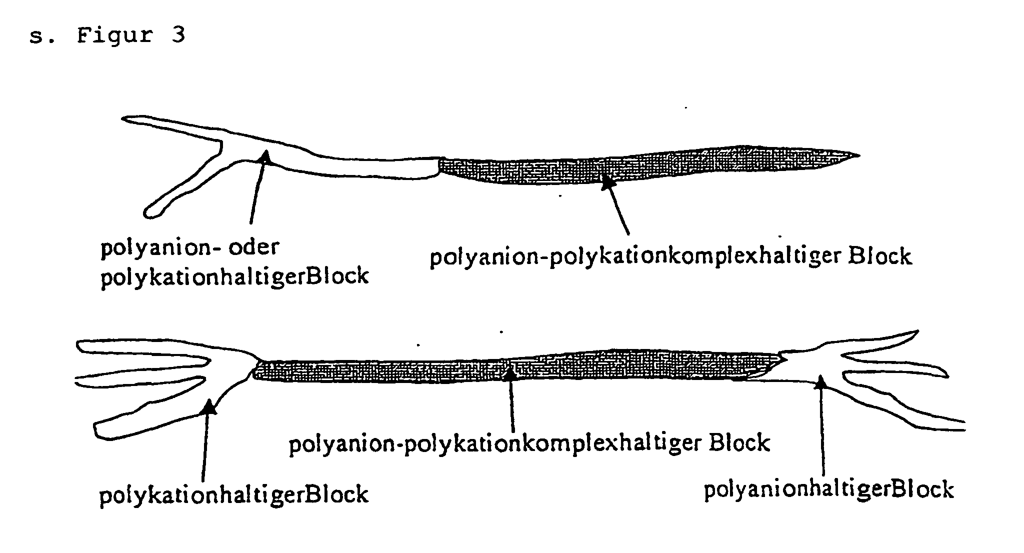

Die faserförmigen polyanion-polykation-komplexhaltigen Gebilde stellen Verstärkungsstoffe dar, die über die auf ihrer Oberfläche fest verankerten polyanionischen und polykationischen Bestandteile bei Zutritt von Wasser oder von wässrigen Lösungen bzw. von wässrigen Sekreten miteinander und/oder mit dem Polyelektrolyt-Feststoffsystem ebenfalls enthaltenen faserförmigen polyanion- und/oder polykationhaltigen Gebilden ionisch vernetzt sind. Durch ihr Vorhandensein geben sie dem durch Solvatation der polyanion-und/oder polykationhaltigen Gebilde entstehenden Gel die für die Wahrung der Integrität erforderliche mechanische Festigkeit.

Für die Herstellung der Polyelektrolyt-Feststoffsysteme sind alle wasserlöslichen anionischen und kationischen PEL, deren Polymerketten aus mindestens 10 Monomerbausteinen bestehen, geeignet. Als anionische PEL kommen vorzugsweise wasserlösliche Salze von Carboxymethylcellulose und/oder Polyacrylsäure und/oder Polymethacrylsäure und/oder Pektin und/oder Carboxymethylchitosan sowie als kationische PEL vorzugsweise wasserlösliche Chitosansalze zum Einsatz.

Das Verhältnis der polyanionischen und der polykationischen Bestandteile in dem Gemisch der faserförmigen polyanion-polykation-komplexhaltigen Gebilde und der polyanion- und/oder polykationhaltigen Gebilde weicht bis zu 98%, vorzugsweise bis zu 80% von dem stöchiometrischen Polyanion-/Polykation-Verhältnis ab.

Die Mischungen der faserförmigen polyanion-polykation-komplexhaltigen Gebilde und der polyanion- und/oder polykationhaltigen Gebilde werden in einer speziellen Ausführung auf oder in Trägern fixiert.

In einer vorteilhaften Ausführung der Erfindung enthalten die Mischungen der faserförmigen polyanion-polykation-komplexhaltigen Gebilde und der polyanion- und/oder polykationhaltigen Gebilde weitere Bestandteile, vorzugsweise Füllstoffe und nichtionische Wirkstoffe, die nicht an der Polyanion-Polykation-Reaktion teilnehmen.

Es ist weiterhin vorteilhaft, wenn die faserförmigen polyanion-polykation-komplexhaltigen Gebilde und die polyanion- und/oder polykationhaltigen Gebilde nieder- und hochmolekulare Bestandteile wie Silbersalze oder Wirkstoffe mit ionischen Gruppen oder Gelatine, die durch Salzbindungen an ein oder mehrere polyanion-polykation-komplexhaltige Gebilde und polyanion- und/oder polykationhaltige Gebilde gebunden sind, enthalten.

Es ist weiterhin vorteilhaft, wenn die Verteilung der polyanion-polykation-komplexhaltigen Gebilde und der polyanion- und/oder polykationhaltigen Gebilde in den aus den erfindungsgemäßen Polyelektrolyt-Feststoffsystemen geformten Flocken, Flockenverbunden, Vliesen oder Papier nicht homogen ist, sondern diese gezielt herbeigeführt wird, vorzugsweise in Schichten.

Weiterhin betrifft die Erfindung ein Verfahren zur Herstellung eines Polyelektrolyt-Feststoffsystems, bei dem für die Herstellung der Mischung der faserförmigen polyanion-polykation-komplexhaltigen Gebilde, die neben einem ionisch vernetzten Kern oder Block noch polyanion- und/oder polykation-haltige Bestandteile enthalten, wässrige Lösungen der anionischen und kationischen Polyelektrolyte gemeinsam durch ein Mischungsrohr hindurchfließen und die Mischungen faserförmiger polyanion-polykation-komplexhaltiger Gebilde und polyanion- und/oder polykation-haltiger Gebilde durch mechanische Verspinnung der vorgemischten wässrigen Lösungen der anionischen und kationischen Polyelektrolyten unter Verwendung wasserziehender Fällmedien erfolgt. Für die Herstellung der erfindungsgemäßen Polyelektrolyt-Feststoffsysteme, bestehend aus Gemischen faserförmiger polyanion-polykation-komplexhaltiger Gebilde und polyanion-und/oder polykationhaltiger Gebilde kommen vorwiegend wässrige Lösungen polyanionischer und polykationischer PEL zur Anwendung. Diese Lösungen können bei einer geeigneten Temperatur unter Anwendung von Druck durch Rohrleitungen gefördert und durch Düsen gedrückt werden. Um homogene Lösungen zu erhalten, ist es in einigen Fällen vorteilhaft, vorzugsweise pulverförmige PEL in einem mit Wasser mischbaren PEL-Nichtlösungsmittel zu dispergieren und diese Dispersion für die Lösungsherstellung zu verwenden. Als Nichtlösemittel werden vorzugsweise solche Substanzen eingesetzt, die bei der Herstellung der Mischungen der faserförmigen polyanion-polykation-komplexhaltigen Gebilde und der polyanion-und/oder polykationhaltigen Gebilde als Fällmittel Verwendung finden. Es kann weiterhin vorteilhaft sein, wenn der Löseprozess vorzeitig abgebrochen wird und die entstandenen wässrigen Lösungen noch ungelöste PEL-Partikel oder PEL-Gelteilchen enthalten. Es kann ebenfalls vorteilhaft sein, von der unlöslichen Säure- oder Basenform des PEL auszugehen und diese durch Zugabe geeigneter Basen oder Säure in Lösung zu bringen.

Voraussetzung für die Entstehung der in den vorstehenden Bildern schematisch dargestellten polyanion-polykation-komplexhaltigen Gebilde ist der gemeinsame Durchfluss der PEL-Ausgangslösungen durch ein Mischungsrohr, wobei an den Grenzflächen der Lösungen die anionischen und die kationischen PEL miteinander unter Ausbildung eines Polyanion-Polykation-Komplexfilms reagieren. Mit den in dem Mischungsrohr gegebenenfalls befindlichen statischen und/oder dynamischen Mischerelementen kann die Größe der Grenzfläche und damit der Gehalt an Polyanion-Polykation-Komplexfilm wesentlich beeinflusst werden.

Die Herstellung der Mischungen faserförmiger polyanion-polykation-komplexhaltiger und polyanion und/oder polykationhaltiger Gebilde erfolgt durch mechanische Verspinnung der polyanion-polykation-komplexfilmhaltigen vorgemischten wässrigen Lösungen von anionischen und kationischen Polyelektrolyten unter Verwendung wasserziehender Fällmedien in einem intensiven Scherfeld. Als Scherfeldgeneratoren werden vorzugsweise nach dem Rotor/Statorprinzip arbeitende Dispergatoren verwendet. Bei der mechanischen Verspinnung werden aus der Polyanionlösung faserförmige ausschließlich polyanionhaltige Gebilde, aus der Polykationlösung faserförmige ausschließlich polykationhaltige Gebilde und aus dem Polyanion-Polykation-Komplexfilm sowie den daran anhaftenden Polyanion- und Polykationlösungen die faserförmigen polyanion-polykation-komplexhaltigen Gebilde geformt. Die faserförmigen Gebilde verlassen dispergiert im Fällmittel den Scherfeldgenerator.

Das Polyanion-Polykation-Verhältnis sowie die Turbulenz im Mischungsrohr kann so gewählt werden, dass bei der mechanischen Verspinnung neben den faserförmigen polyanion-polykation-komplexhaltigen Gebilden nur faserförmige ausschließlich polyanionhaltige oder ausschließlich polykationhaltige Gebilde entstehen.

Bei der mechanischen Verspinnung geht der größte Teil des Wassers aus den PEL-Lösungen in das Fällmittel über. Um während des Trockungsprozesses ein Zusammenschmelzen der Gebilde durch partielles Lösen der PEL zu vermeiden, wird das in und um die faserförmigen Gebilde befindliche Restwasser durch azeotrope Destillation vorsichtig entfernt. Das Wasser ziehende Fällmittel enthält mindestens einen Bestandteil, der mit dem Wasser ein Azeotrop bildet.

Schließlich betrifft die Erfindung noch einen Wundverband, bei dem ein Polyelektrolyt-Feststoffsystem wie zuvor beschrieben Verwendung findet. Es kann dabei vorgesehen sein, dass die Verbände in Form von Pulvern, Tamponadestreifen oder flächigen Kompressen vorliegen.

Insbesondere können die Verbände einen Wassergehalt von 0 bis 20 Gew.-%, insbesondere von 0,5 bis 5 Gew.-% aufweisen.

Bei einem Wundverband in Form von flächigen Kompressen können die Polyelektrolyt-Feststoffsysteme auf Trägermaterialien aufgebracht sein. Alternativ können die Polyelektrolyt-Feststoffsysteme zwischen zwei oder mehreren Trägermaterialien einlaminiert sein. Als Trägermaterialien kommen Filme, Textilien, Folien, Vliesstoffe, Papier oder Schäume in Frage.

Im folgenden soll die Erfindung anhand von vier Beispielen näher erläutert werden:

### Beispiel 1:

In einem Becherglas werden in 900 g Wasser 100 g pulverförmiges Carboxymethylcellulose-Natriumsalz mittelviskos der Firma Fluka in kleinen Portionen eingetragen und so lange intensiv gerührt, bis sich eine klare hochviskose Lösung (Lösung A) gebildet hat. In einem zweiten Becherglas werden 450 g Wasser und 50 g Chitosan niedrigviskos der Firma Fluka mit einem Ultraturax zu einem frei Brei zermahlen und homogenisiert. Unter Rühren wird so lange langsam 10%-ige Salzsäure zugesetzt, bis das Chitosan als Chitosanhydrochlorid in Lösung (Lösung B) gegangen ist. Die Menge des zugegebenen HCl ist so zu bemessen, dass der pH-Wert der entstandenen Lösung 5,5 beträgt.

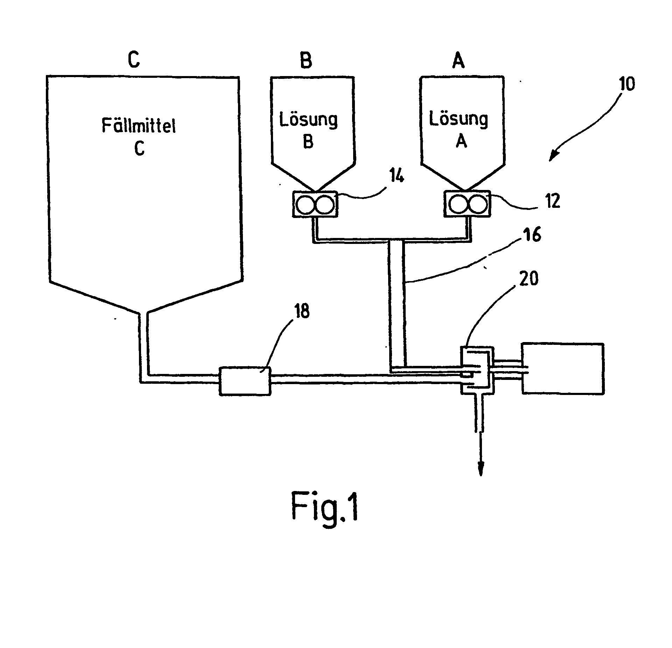

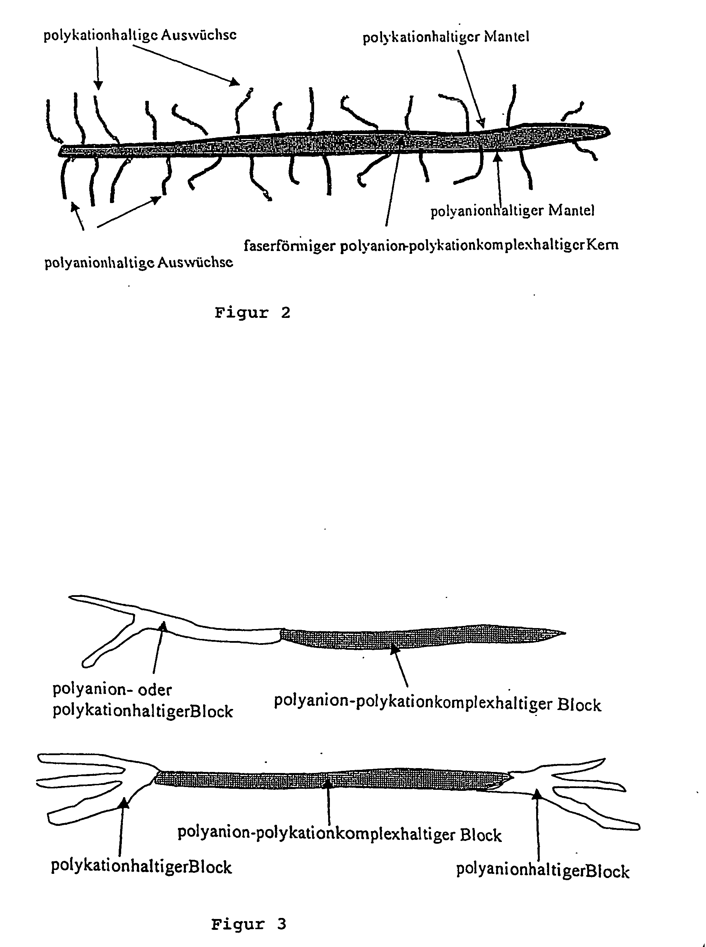

Lösung A und Lösung B werden gemeinsam in der in Figur 1 skizzierten Vorrichtung 10 zu faserförmigen polyanion-polykation-komplexhaltigen Gebilden und polyanion- und polykationhaltigen Gebilden versponnen.

Hierzu werden Lösung A und Lösung B in die Vorratsbehälter A und B gegeben und über separate Zahnradpumpen 12, 14 in einem Verhältnis von 2:1 und einem Durchsatz von 60 g/min. dem Mischrohr 16 zugeführt, welches sie gemeinsam unter Ausbildung von Polyanion-Polykation-Komplexgrenzflächenfilmen passieren. Das im Mischrohr 16 entstandene Gemisch wird gleichzeitig mit 340 g/min. eines 2:1 n-Propanol/Aceton-Gemisches, welches sich in dem Fällmittelbehälter C befindet und mit einer Membranpumpe 18 dosiert wird, in das Rotationszentrum des Spinnkopfes 20 (Ultraturax UTL 25 der Firma IKA) geführt und bei einer Drehzahl von 24 000 U/min. mechanisch versponnen. Den Scherfeldgenerator verlassen pro Minute 400 g PEL-Dispersion.

Die Dispersion der faserförmigen Gebilde wird in 200 g-Portionen über einen mit Filterpapier belegten Büchnertrichter (Durchmesser 100 mm) abgesaugt und das dem Filterkuchen noch anhaftende n-Propanol/Aceton/Wassergemisch bei 60°C in einem mit einer Absaugung versehenen explosionsgeschützten Trockenschrank entfernt.

Die auf diese Weise erhaltenen Vliese nehmen mehr als das Zehnfache ihres Gewichtes an 0,09%-iger Kochsalzlösung auf und sind in diesem Zustand flexibel und mechanisch belastbar.

### Beispiel 2:

a) Herstellung einer Dispersion faserförmiger Gebilde gemäß Beispiel 1
b) Herstellung einer Dispersion faserförmiger Gebilde gemäß Beispiel 1, jedoch beträgt das zur Verspinnung gebrachte Verhältnis der Lösungen von Carboxymethylcellulose-Natrium und Chitosanhydrochlorid 1:9.

Zur Vliesbildung werden zuerst 20 g der nach B hergestellten Dispersion faserförmiger PEL-Gebilde über einen mit Filterpapier gelegten Büchnertrichter (Durchmesser 100 mm) abgesaugt und anschließend 180 g der nach a) hergestellten Dispersion faserförmiger PEL-Gebilde vorsichtig aufgegossen und ebenfalls abgesaugt. Die Entfernung des noch anhaftenden Propanol/Aceton/Wassergemisches erfolgt wie in Beispiel 1. Es werden zweischichtige Vliese erhalten, die mehr als das Zehnfache ihres Gewichtes an 0,9%-iger Kochsalzlösung aufnehmen und in diesem Zustand flexibel und mechanisch belastbar sind.

### Beispiel 3:

In einem Becherglas werden 895 g Wasser und 5 g Zellstoff mit einem Ultraturax aufgeschlagen und zu einer feinteiligen Dispersion vermahlen. Anschließend wird 50 g pulverförmiges Carboxymethylcellulose-Natriumsalz hochviskos der Firma Fluka in 50 g eines 2:1 Gemisches aus n-Propanol und Aceton aufgeschlämmt und die entstandene Dispersion unter intensivem Rühren in die Zellstoffdispersion eingetragen. Es wird so lange gerührt, bis sich eine hochviskose Zellstoffdispersion (Lösung A) gebildet hat. In einem zweiten Becherglas werden 475 g Wasser und 25 g Chitosan hochviskos der Firma Fluka mit einem Ultraturax zu einem feinen Brei zermahlen und homogenisiert. Unter Rühren wird so lange langsam 10%-ige Salzsäure zugesetzt, bis das Chitosan als Chitosanhydrochlorid in Lösung (Lösung B) gegangen ist. Die Menge der zugegebenen HCl ist so zu bemessen, dass der pH-Wert der entstandenen Lösung 5,5 beträgt.

Die Herstellung der PEL-Dispersion und ihre Verarbeitung erfolgt wie in Beispiel 1. Auf diesem Wege können Vliese hergestellt werden, in denen neben den faserförmigen polyanionen-polykationen-komplexhaltigen Gebilden und polyanion- und/oder polykationhaltigen Gebilden Zellstoffstützfasern enthalten sind. Nach der Quellung in 0,9%-iger Kochsalzlösung entsteht ein flexibler und mechanisch belastbarer Gelverbund.

### Beispiel 4:

Die in Beispiel 1 hergestellten Vliese werden mit einer Prallmühle MF 10 basic Sieblochgröße 2 mm der Firma IKA zu Flocken zerfasert. 0,5 g dieser Flocken werden in eine Form von 2x2 cm eingefüllt und zu einem lockeren Flockenverbund leicht verdichtet, der mehr als das Zehnfache seines Gewichtes an 0,9%-iger Kochsalzlösung aufnehmen kann und in diesem Zustand flexibel und mechanisch belastbar ist.

## Patentansprüche

1. Polyelektrolyt-Feststoffsystem, insbesondere in Form von Flocken, Flockenverbunden, Vliesen oder Papier, **dadurch gekennzeichnet, dass** dieses eine Mischung aus faserförmigen polyanion-polykation-komplexhaltigen Gebilden, die neben einem ionisch vernetzten Kern oder Block noch polyanion- und/oder polykationhaltige Bestandteile enthalten, und aus polyanion- und/oder polykationhaltigen Gebilden umfasst, wobei die jeweiligen anionischen und kationischen Polyelektrolyte wasserlösliche Polyelektrolyte sind, die Polymerketten mit mindestens 10 Monomerbausteinen besitzen und wobei das Verhältnis der polyanionischen und der polykationischen Bestandteile in dem Gemisch der faserförmigen polyanion-polykation-komplexhaltigen Gebilde und der polyanion- und/oder polykation-haltigen Gebilde bis zu 98 % vom stöchiometrischen Verhältnis Polyanion zu Polykation abweicht.

2. Polyelektrolyt-Feststoffsystem nach Anspruch 1, **dadurch gekennzeichnet, dass** als anionische Polyelektrolyte wasserlösliche Salze, insbesondere von Carboxymethlycellulose und/oder Polyacrylsäure und/oder Polymethacrylsäure und/oder Pektin und/oder Carboxymethylchitosan sowie als kationische Polyelektrolyte, insbesondere wasserlösliche Chitosansalze, dienen.

3. Polyelektrolyt-Feststoffsystem nach Anspruch 1 oder 2, **dadurch gekennzeichnet, dass** das Verhältnis der polyanionischen und der polykationischen Bestandteile in dem Gemisch der faserförmigen polyanion-polykation-komplexhaltigen Gebilde und der polyanion- und/oder polykationhaltigen Gebilde bis zu 98%, vorzugsweise bis zu 80%, vom stöchiometrischen Polyanion-/Polykation-Verhältnis abweicht.

4. Polyelektrolyt-Feststoffsystem nach einem der vorangehenden Ansprüche, **dadurch gekennzeichnet, dass** die Mischungen der faserförmigen polyanion-polykation-komplexhaltigen Gebilde und der polyanion- und/oder polykationhaltigen Gebilde auf oder in Trägern fixiert sind.

5. Polyelektrolyt-Feststoffsystem nach einem der vorangehenden Ansprüche, **dadurch gekennzeichnet, dass** die Mischung der faserförmigen polyanion-polykation-komplexhaltigen Gebilde und der polyanion- und/oder polykationhaltigen Gebilde weitere Bestandteile, die nicht an der Polyanion-Polykation-Reaktion teilnehmen, enthält.

6. Polyelektrolyt-Feststoffsystem nach Anspruch 5, **dadurch gekennzeichnet, dass** ein oder mehrere der Bestandteile, die nicht an der Polyanion-Polykation-Reaktion teilnehmen, Fasern zur mechanischen Verstärkung der Systeme darstellen.

7. Polyelektrolyt-Feststoffsystem nach einem der vorangehenden Ansprüche, **dadurch gekennzeichnet, dass** die Mischung der faserförmigen polyanion-polykation-komplexhaltigen Gebilde und der polyanion- und/oder polykationhaltigen Gebilde nieder- und hochmolekulare Bestandteile enthält, die durch Salzbindungen an ein oder mehrere polyanion-polykation-komplexhaltige Gebilde und ein oder mehrere polyanion- und/oder polykationhaltige Gebilde gebunden sind, insbesondere Silbersalze, Wirkstoffe mit ionischen Gruppen oder Gelatine.

8. Polyelektrolyt-Feststoffsystem nach einem der vorangehenden Ansprüche, **dadurch gekennzeichnet, dass** die Verteilung der polyanion-polykation-komplexhaltigen Gebilde und der polyanion- und/oder polykationhaltigen Gebilde nicht homogen ist, sondern insbesondere eine schichtenartige Verteilung besteht.

9. Verfahren zur Herstellung eines Polyelektrolyt-Feststoffsystems nach einem der vorangehenden Ansprüche, **dadurch gekennzeichnet, dass** für die Herstellung der Mischung der faserförmigen polyanion-polykation-komplexhaltigen Gebilde, die neben einem ionisch vernetzten Kern oder Block noch polyanion- und/oder polykation-haltige Bestandteile enthalten, wässrige Lösungen der anionischen und kationischen Polyelektrolyte gemeinsam durch ein Mischungsrohr hindurchfließen und die Mischungen faserförmiger polyanion-polykation-komplexhaltiger Gebilde und polyanion- und/oder polykation-haltiger Gebilde durch mechanische Verspinnung der vorgemischten wässrigen Lösungen der anionischen und kationischen Polyelektrolyten unter Verwendung wasserziehender Fällmedien erfolgt.

10. Verfahren nach Anspruch 9, **dadurch gekennzeichnet, dass** für die Ausbildung der polyanion-polykation-komplexhaltigen Gebilde ein gemeinsamer Durchfluss der wässrigen Lösung von anionischen und kationischen Polyelektrolyten durch ein Mischungsrohr verwendet wird.

11. Verfahren nach Anspruch 10, **dadurch gekennzeichnet, dass** sich in dem Mischungsohr statische und/oder dynamische Mischerelemente befinden.

12. Verfahren nach einem der vorstehenden Ansprüche, **dadurch gekennzeichnet, dass** die Mischungen faserförmiger polyanion-polykation-komplexhaltiger Gebilde und polyanion- und/oder polykationhaltiger Gebilde durch mechanische Verspinnung vorgemischter wässriger Lösungen von anionischen und kationischen Polyelektrolyten vorzugsweise in einem nach dem Rotor-Statorprinzip arbeitenden Dispergator unter Verwendung wasserziehender Fällmedien erfolgt.

13. Verfahren nach Anspruch 12, **dadurch gekennzeichnet, dass** die wasserziehenden Fällmedien mindestens einen Bestandteil enthalten, der mit Wasser ein Azeotrop bildet.

14. Wundverband, **dadurch gekennzeichnet, dass** ein Polyelektrolyt-Feststoffsystem nach einem der Ansprüche 1 bis 9 verwendet wird.

15. Wundverband nach Anspruch 14, **dadurch gekennzeichnet, dass** die Verbände in Form von Pulvern, Tamponadestreifen oder flächigen Kompressen vorliegen.

16. Wundverband nach Anspruch 14 oder 15, **dadurch gekennzeichnet, dass** die Verbände einen Wassergehalt von 0 bis 20 Gew.-%, insbesondere von 0,5 bis 5 Gew.-% aufweisen.

17. Wundverband nach einem der vorstehenden Ansprüche in Form von flächigen Kompressen, **dadurch gekennzeichnet, dass** die Polyelektrolyt-Feststoffsysteme auf Trägermaterialien aufgebracht sind.

18. Wundverband nach einem der vorstehenden Ansprüche in Form von flächigen Kompressen, **dadurch gekennzeichnet, dass** die Polyelektrolyt-Feststoffsysteme zwischen zwei oder mehreren Trägermaterialien einlaminiert sind.

19. Wundverband nach Anspruch 17 oder 18, **dadurch gekennzeichnet, dass** die Trägermaterialien als Filme, Textilien, Folien, Vliesstoffe, Papier oder Schäume vorliegen.

## Claims

1. Polyelectrolyte solid system, in particular in the form of flocks, flock composites, non-wovens or paper, **characterised in that** it comprises a mixture of fibrous entities containing polyanion-polycation complexes, which entities, in addition to an ionically crosslinked core or block, also contain components containing polyanions and/or polycations, and of entities containing polyanions and/or polycations, the respective anionic and cationic polyelectrolytes being water-soluble polyelectrolytes, which have polymer chains comprising at least 10 monomer units, and the ratio of the polyanionic and polycationic components in the mixture of the fibrous entities containing polyanion-polycation complexes and of the entities containing polyanions and/or polycations differing by up to 98 % from the stoichiometric polyanion-polycation ratio.

2. Polyelectrolyte solid system according to claim 1, **characterised in that** water-soluble salts, in particular of carboxymethyl cellulose and/or polyacrylic acid and/or polymethylacrylic acid and/or pectin and/or carboxymethyl chitosan, act as anionic polyelectrolytes and as cationic polyelectrolytes, in particular water-soluble chitosan salts.

3. Polyelectrolyte solid system according to either claim 1 or claim 2, **characterised in that** the ratio of polyanionic and polycationic components in the mixture of the fibrous entities containing polyanion-polycation complexes and of the entities containing polyanions and/or polycations differs by up to 98 %, preferably up to 80 %, from the stoichiometric polyanion-polycation ratio.

4. Polyelectrolyte solid system according to any one of the preceding claims, **characterised in that** the mixtures of the fibrous entities containing polyanion-polycation complexes and of the entities containing polyanions and/or polycations are fixated in or on carriers.

5. Polyelectrolyte solid system according to any one of the preceding claims, **characterised in that** the mixture of the fibrous entities containing polyanion-polycation complexes and of the entities containing polyanions and/or polycations contains further components, which are not part of the polyanion-polycation reaction.

6. Polyelectrolyte solid system according to claim 5, **characterised in that** one or more of the components, which are not part of the polyanion-polycation reaction, form fibres for the mechanical reinforcement of the systems.

7. Polyelectrolyte solid system according to any one of the preceding claims, **characterised in that** the mixture of the fibrous entities containing polyanion-polycation complexes and of the entities containing polyanions and/or polycations contains low-molecular and high-molecular components, which are bound by salt bonds to one or more entities containing polyanion-polycation complexes and to one or more entities containing polyanions and/or polycations, in particular silver salts, active ingredients comprising ionic groups or gelatine.

8. Polyelectrolyte solid system according to any one of the preceding claims, **characterised in that** the distribution of the entities containing polyanion-polycation complexes and of the entities containing polyanions and/or polycations is not homogeneous, but is, in particular, a stratified distribution.

9. Process for producing a polyelectrolyte solid system according to any one of the preceding claims, **characterised in that** for producing the mixture of the fibrous entities containing polyanion-polycation complexes, which entities, in addition to an ionically crosslinked core or block, also contain components containing polyanions and/or polycations, aqueous solutions of the anionic and cationic polyelectrolytes flow through a mixing tube together and the mixing of fibrous entities containing polyanion-polycation complexes and of entities containing polyanions and/or polycations takes place by mechanical spinning of the premixed aqueous solutions of the anionic and cationic polyelectrolytes with the use of hydrophilic precipitants.

10. Process according to claim 9, **characterised in that** for the formation of the entities containing polyanion-polycation complexes, joint flow of the aqueous solution of anionic and cationic polyelectrolytes through a mixing tube is employed.

11. Process according to claim 10, **characterised in that** static and/or dynamic mixing elements are present in the mixing tube.

12. Process according to any one of the preceding claims, **characterised in that** the mixing of fibrous entities containing polylanion-polycation complexes and entities containing polyanions and/or polycations takes place by mechanical spinning of premixed aqueous solutions of anionic and cationic polyelectrolytes, preferably in a shear mixer working on the rotor-stator principle, with the use of hydrophilic precipitants.

13. Process according to claim 12, **characterised in that** the hydrophilic precipitants contain at least one component which forms an azeotrope with water.

14. Wound dressing, **characterised in that** a polyelectrolyte solid system according to any one of claims 1 to 9 is used.

15. Wound dressing according to claim 14, **characterised in that** the dressings are available in the form of powders, strips for tamponage or flat compresses.

16. Wound dressing according to either claim 14 or claim 15, **characterised in that** the dressings have a water content of 0 to 20 % by weight, in particular of 0.5 to 5 % by weight.

17. Wound dressing according to any one of the preceding claims in the form of flat compresses, **characterised in that** the polyelectrolyte solid systems are applied to carrier materials.

18. Wound dressing according to any one of the preceding claims in the form of flat compresses, **characterised in that** the polyelectrolyte solid systems are laminated between two or more carrier materials.

19. Wound dressing according to either claim 17 or claim 18, **characterised in that** the carrier materials are available as films, textiles, plastic sheets, non-woven materials, paper or foams.

## Revendications

1. Système à polyélectrolyte solide, notamment sous forme de flocons, de composites floconneux, de non-tissés ou de papier, **caractérisé en ce que** celui-ci comporte un mélange composé de produits sous forme fibreuse contenant un complexe polyanions-polycations, lesquels comportent, outre un noyau ou bloc réticulé ionique, également des composants à base de polyanions et/ou de polycations, et de produits à base de polyanions et/ou de polycations, les polyélectrolytes anioniques et cationiques concernés étant des polyélectrolytes solubles dans l'eau qui possèdent des chaînes polymères ayant au moins 10 éléments monomères et le rapport entre les composants polyanioniques et les composants polycationiques dans le mélange composé des produits sous forme fibreuse contenant un complexe polyanions-polycations et des produits à base de polyanions et/ou de polycations s'écarte jusqu'à 98 % du rapport stcechiométrique polyanions/polycations.

2. Système à polyélectrolyte solide selon la revendication 1, **caractérisé en ce que** l'on utilise, comme polyélectrolytes anioniques, des sels solubles dans l'eau, en particulier de carboxyméthylcellulose et/ou d'acide polyacrylique et/ou d'acide polyméthacrylique et/ou de pectine et/ou de carboxyméthylchitosane et, comme polyélectrolytes cationiques, en particulier des sels de chitosane solubles dans l'eau.

3. Système à polyélectrolyte solide selon la revendication 1 ou 2, **caractérisé en ce que** le rapport entre les composants polyanioniques et les composants polycationiques dans le mélange composé des produits sous forme fibreuse contenant un complexe polyanions-polycations et des produits à base de polyanions et/ou de polycations s'écarte jusqu'à 98 %, de préférence jusqu'à 80 %, du rapport stoechiométrique polyanions/polycations.

4. Système à polyélectrolyte solide selon l'une des revendications précédentes, **caractérisé en ce que** les mélanges composés des produits sous forme fibreuse contenant un complexe polyanions-polycations et des produits à base de polyanions et/ou de polycations sont fixés sur ou dans des substrats.

5. Système à polyélectrolyte solide selon l'une des revendications précédentes, **caractérisé en ce que** le mélange composé des produits sous forme fibreuse contenant un complexe polyanions-polycations et des produits à base de polyanions et/ou de polycations comporte d'autres composants qui ne participent pas à la réaction polyanions/polycations.

6. Système à polyélectrolyte solide selon la revendication 5, **caractérisé en ce qu'**un ou plusieurs des composants qui ne participent pas à la réaction polyanions/polycations sont des fibres destinées à renforcer mécaniquement les systèmes.

7. Système à polyélectrolyte solide selon l'une des revendications précédentes, **caractérisé en ce que** le mélange composé des produits sous forme fibreuse contenant un complexe polyanions-polycations et des produits à base de polyanions et/ou de polycations comporte des composants de faible poids moléculaire et de poids moléculaire élevé qui sont combinés par des liaisons salines avec un ou plusieurs produits contenant un complexe polyanions-polycations et avec un ou plusieurs produits à base de polyanions et/ou de polycations, en particulier des sels d'argent, des agents ayant des groupes ioniques ou de la gélatine.

8. Système à polyélectrolyte solide selon l'une des revendications précédentes, **caractérisé en ce que** la répartition des produits contenant un complexe polyanions-polycations et des produits à base de polyanions et/ou de polycations n'est pas homogène, mais est en particulier une répartition par couches.

9. Procédé de fabrication d'un système à polyélectrolyte solide selon l'une des revendications précédentes, **caractérisé en ce que**, pour la fabrication du mélange composé des produits sous forme fibreuse contenant un complexe polyanions-polycations, lesquels comportent, outre un noyau ou bloc réticulé ionique, également des composants à base de polyanions et/ou de polycations, des solutions aqueuses des polyélectrolytes anioniques et cationiques traversent ensemble un tube de mélange et les mélanges composés de produits sous forme fibreuse contenant un complexe polyanions-polycations et de produits à base de polyanions et/ou de polycations sont réalisés par filage mécanique des solutions aqueuses pré-mélangées des polyélectrolytes anioniques et cationiques, en utilisant des précipitants absorbant l'eau.

10. Procédé selon la revendication 9, **caractérisé en ce que**, pour la formation des produits contenant un complexe polyanions-polycations, on utilise un passage simultané de la solution aqueuse de polyélectrolytes anioniques et cationiques à travers un tube de mélange.

11. Procédé selon la revendication 10, **caractérisé en ce que**, dans le tube de mélange, il y a des éléments mélangeurs statiques et/ou dynamiques.

12. Procédé selon l'une des revendications précédentes, **caractérisé en ce que** les mélanges composés de produits sous forme fibreuse contenant un complexe polyanions-polycations et de produits à base de polyanions et/ou de polycations sont réalisés par filage mécanique de solutions aqueuses pré-mélangées de polyélectrolytes anioniques et cationiques, de préférence dans un disperseur fonctionnant suivant le principe du rotor-stator, en utilisant des précipitants absorbant l'eau.

13. Procédé selon la revendication 12, **caractérisé en ce que** les précipitants absorbant l'eau contiennent au moins un composant qui forme un azéotrope avec l'eau.

14. Pansement, **caractérisé en ce qu'**un système à polyélectrolyte solide selon l'une des revendications 1 à 9 est utilisé.

15. Pansement selon la revendication 14, **caractérisé en ce que** les pansements se présentent sous forme de poudres, de bandes de tamponnement ou de compresses minces.

16. Pansement selon la revendication 14 ou 15, **caractérisé en ce que** les pansements présentent une teneur en eau de 0 à 20 % en poids, en particulier de 0,5 à 5 % en poids.

17. Pansement selon l'une des revendications précédentes sous forme de compresses minces, **caractérisé en ce que** les systèmes à polyélectrolyte solide sont appliqués sur des matières de substrat.

18. Pansement selon l'une des revendications précédentes sous forme de compresses minces, **caractérisé en ce que** les systèmes à polyélectrolyte solide sont laminés entre deux ou plusieurs matières de substrat.

19. Pansement selon la revendication 17 ou 18, **caractérisé en ce que** les matières de substrat se présentent sous forme de films, textiles, feuilles, non-tissés, papier ou mousses.
